# EUROPÄISCHE PATENTSCHRIFT

(11) **EP 0 871 453 B1**
(45) Veröffentlichungstag und Bekanntmachung des Hinweises auf die Patenterteilung: **21.05.2003**
(21) Anmeldenummer: 96913497.2
(22) Anmeldetag: 04.04.1996
(51) Int. Cl.: A61K 31/585, A61K 31/57, A61K 31/565, A61K 31/47

(54) **PHARMAZEUTISCHES KOMBINATIONSPRÄPARAT ZUR HORMONALEN KONTRAZEPTION**
COMBINED HORMONAL CONTRACEPTION PHARMACEUTICAL PREPARATION
PREPARATION PHARMACEUTIQUE COMBINEE DE CONTRACEPTION HORMONALE

(30) Priorität: 08.04.1995 DE 19513662
(43) Veröffentlichungstag der Anmeldung: 21.10.1998
(73) Patentinhaber: SCHERING AKTIENGESELLSCHAFT, 13342 Berlin (DE)
(72) Erfinder: SPONA, Jürgen, 1130 Wien (AT); DÜSTERBERG, Bernd, 12307 Berlin (DE)
(86) Internationale Anmeldenummer: EP9601529
(87) Internationale Veröffentlichungsnummer: WO96032114

(56) Entgegenhaltungen:
- EP-A- 0 163 490
- EP-A- 0 499 348
- WO-A-92/07589
- WO-A-95/07081
- WO-A-95/08329
- DE-A- 1 939 636
- DE-A- 4 344 405
- DE-A- 4 344 462
- DE-A- 4 411 585
- US-A- 3 502 772
- US-A- 4 921 843

## Beschreibung

Die vorliegende Erfindung betrifft ein pharmazeutisches Kombinationspräparat mit zwei in einer Verpackungseinheit räumlich getrennt konfektionierten, zur zeitlich sequentiellen oralen Verabreichung bestimmten Hormonkoniponenten, die jeweils aus einer Anzahl räumlich getrennt und einzeln entnehmbar in der Verpackungseinheit untergebrachten täglichen Dosierungseinheiten bestehen, wobei eine erste der Hormonkomponenten als hormonellen Wirkstoff in Kombination ein Estrogen- und in mindestens zur Ovulationshemmung ausreichender Dosierung ein Gestagenpräparat in entweder ein- oder mehrstufiger Ausbildung und die zweite Hormonkomponente als hormonellen Wirkstoff lediglich ein Estrogenpräparat enthält, wobei die erste Hormonkomponente 23 oder 24 und die zweite Hormonkomponente 4, 3 oder 2 Tageseinheiten umfaßt und sich zwischen diesen beiden Hormonkomponenten 2 oder 1 wirkstofffreie Tageseinheiten (Placebos) befinden oder 2 oder 1 Blindpillen-Tage angezeigt sind und die Gesamtzahl der Hormontageseinheiten sowie der wirkstofffreien Tageseinheiten beziehungsweise der Blindpillen-Tage gleich der Gesamtzahl der Tage des gewünschten, mindestens aber 28 Tage langen, Zyklus ist, sowie eine entsprechende, dieses Kombinationspräparat enthaltende Packung.

Orale Kontrazeptiva in Form von Kombinationspräparaten sind als sogenannte Einphasenpräparate seit 1960 bekannt. Diese Präparate bestehen aus 21 wirkstoffhaltigen Dosierungseinheiten und 7 wirkstofffreien Tabletten oder Dragees. Die tägliche Dosierungseinheit ist aus einem Estrogen und Gestagen zusammengesetzt. In Einphasenpräraraten ist die täglich zu verabreichende Dosis der aktiven Stoffe in jeder Dosierungseinheit gleich hoch. Wenn die täglich zu verabreichende Dosis der aktiven Bestandteile in den einzelnen Dosierungseinheiten in einzelnen Abschnitten über den Verabreichungszyklus unterschiedlich ist, handelt es sich um sogenannte Mehrphasenpräparate. Als besonders namhafter Vertreter sei Triquilar® genannt (DE-A 23 65 103).

Durch die Entwicklung neuer, wirksamerer Gestagene als die in den ersten oralen Kontrazeptiva enthaltenen, konnte die tägliche Gestagendosierung kontinuierlich verringert werden. Auch die tägliche Estrogendosierung konnte gesenkt werden, obwohl als Estrogen in hormonalen Kontrazeptiva nach wie vor meist Ethinylestradiol enthalten ist.
Bei der Entwicklung neuer, verbesserter oraler Kontrazeptiva standen (und stehen) folgende drei Gesichtspunkte im Vordergrund:
Es soll
(1) die kontrazeptive Sicherheit,
(2) eine gute Zykluskontrolle, d.h. geringe Inzidenz an Zwischenblutungen und
(3) ein Minimum an unerwünschten Nebenwirkungen
gewährleistet sein.
Die kontrazeptive Sicherheit wird vor allem durch die Gestagenkomponente bewirkt. Deren tägliche Dosierungsmenge enspricht jeweils mindestens der Grenzdosis, die für das betreffende Gestagen zur Ovulationshemmung als erforderlich angesehen wird. Das in Kombinationspräparaten als Estrogen meistens verwendete Ethinylestradiol soll den ovulationshemmenden Effekt des Gestagens erhöhen und vor allem die Zyklusstabilität gewährleisten. Die tägliche Dosis bei alleiniger Verabreichung des Ethinylestradiols, die für eine Hemmung der Ovulation verwendet werden muss, beträgt 100 µg.

Kombinationspräparate mit der jüngsten Generation von Gestagenen sind z.B. die Einphasenpräparate Femovan (DE-PS 2546062) oder Marvelon (DE-OS 2361120).
Als Mehrphasenpräparat, dessen Dosierungseinheiten ein Gestagen der jüngsten Generation, nämlich Gestoden, enthalten, ist beispielsweise Milvane® zu nennen (EP-0 148 724). Bei diesen Dreiphasenpräparaten werden in der ersten Phase zumeist 4-6 Dragees verabreicht, in der jedes Dragee eine Estrogenmenge in geringer Dosis und ein Gestagen in geringer Dosis enthält. In der zweiten Phase von 4-6 Dragees enthält jede Dosierungseinheit ein Estrogen mit gleicher oder gering angehobener, maximal bis auf das 2 fache gesteigerte Dosis und ein Gestagen mit gleicher oder gering angehobener, maximal auf das 1.5 fach gesteigerte Dosis. In einer dritten Phase von 9-11 Einheiten enthält jedes Dragee ein Estrogen mit gleicher oder wieder gesenkter, maximal auf den Ausgangswert erniedrigter Dosis und ein Gestagen mit weiter angehobener, maximal auf das 3 fache des Ausgangswertes gesteigerter Dosis. Daran schliessen sich 7 pillenfreie Tage an.
Neuerdings sind auch mehrphasische Kombinationspräparate vorgeschlagen worden, die eine verlängerte, d.h. bis zu 24tägige Einnahme wirkstoffhaltiger Dosierungseinheiten im 28tägigen Zyklus vorsehen können. Dabei steigt die tägliche Gestagen-Dosierungsmenge von der ersten über die zweite bis zur dritten Phase entweder an (EP-A 0 491 415) oder sie nimmt ab (EP-A 0 491 438). Zur Vervollständigung des 28 Tage langen Zyklus schließen sich im ersten Fall 4 Blindpillen-Tage, 4 Placebos oder aber 4 ausschließlich gestagenhaltige Dosierungseinheiten oder im zweiten Fall 4 bis 7 Blindpillen-Tage oder 4 bis 7 Placebos an.

Die Entwicklung neuer oraler Kontrazeptiva mit verringerter täglicher Hormondosis hatte zum Ziel, die in epidemologischen Studien beschriebenen Nebenwirkungen zu minimieren. Neuere epidemologische Daten weisen auf einen solchen Trend zur besseren Verträglichkeit niedriger dosierter Präparate hinsichtlich kardiovaskulärer Nebenwirkungen hin.[ Thorogood M, Oral Contraceptives and Cardiovascular Disease: An Epidemiologic Overview; Pharmacoepidemiology and Drug Safety, Vol 2: 3-16 (1993); Gerstman B B, Piper J M, Tomita D K, Ferguson W J, Stadel B V, Lundin F E; Oral Contraceptive Estrogen Dose and the Risk of Deep Venous Thromboembolic Disease, Am J E, Vol. 133, No 1, 32-36 (1991); Lidegaard O, Oral contraception and rist of a cerebral thromboembolic attack: results of a case-control study; BMJ Vol 306, 956-63 (1993); Vessey M, Mant D, Smith A, Yeates D., Oral contraceptives and venous thromboembolism: findings in a large prospective study; BMJ, Vol 292, (1986); Mishell D R, Oral Contraception: Past, Present, and Future Perspectives; Int J Fertil, 36 Suppl., 7 - 18 (1991)].

Ein Zusammenhang zwischen der Höhe der täglichen Estrogendosis und der Häufigkeit kardiovaskulärer Komplikationen wird angenommen.

Das Präparat mit der zur Zeit niedrigst dosierten Estrogenmenge ist als Mercilon® im Handel und enthält 20µg Ethinylestradiol in Kombination mit 150µg Desogestrel in jeder täglichen Dosierungseinheit über 21 Tage mit einem sich anschließenden 7 tägigem pillenfreien Intervall. Die Zykluskontrolle dieses Präparates ist im Vergleich zu Präparaten mit höherer Estrogendosis erwartungsgemäß etwas schlechter. Ein weiteres , klinisch bedeutsames Problem stellt die in mehreren Studien übereinstimmend gemachte Beobachtung einer geringeren ovariellen Suppression des 20µg Ethinylestradiol enthaltenden Präparates dar. Es kommt offensichtlich unter dieser sehr niedrigen Estrogendosis bei vielen Frauen zur Heranreifung von Follikeln, die mit Ultraschalluntersuchungen beziehungsweise Hormonuntersuchungen nachgewiesen werden konnten [Lunell N O, Carlström K, Zador G, Ovulation inhibition with a combined oral contraceptive containing 20 µg ethinylestradiol and 250 µg levonorgestrel; Acta Obstet Gynecol Scand Suppl. 88: 17-21 (1979); Mall-Haefeli M, Werner-Zodrow I, Huber P R, Klinische Erfahrungen mit Mercilon und Marvelon unter besonderer Berücksichtigung der Ovar-Funktion; Geburtsh. und Frauenheilk. 51, 35-38, Georg Thieme Verlag, Stuttgart-New York (1991); Strobel E, Behandlung mit oralen Kontrazeptiva; Fortschr. Med. 110 Jg. Nr. 20 (1992); Letter to Editor, Contraception 45: 519-521 (1992); Teichmann A T, Brill K, Can Dose Reduction of Ethinylestradiol in OCs jeopardize Ovarian Suppression and Cycle Control? Abstract Book, VIIIth World Congress on Human Reproduction, Bali, Indonesia (1993)].

Andere Präparate wurden beschrieben, die einen estrogenen und gestagenen Wirkstoff enthalten und die im allgemeinen über 21 Tage in gleichbleibenden Mengen in jeder einzelnen Dosierungseinheit verabreicht werden, bei denen der Einnahme dieser einen estrogenen und gestagenen Wirkstoff enthaltenden Dosierungseinheiten die Einnahme ausschließlich Estrogen-haltiger Dosierungseinheiten (Ijzerman, US-A 3,502,772; Pasquale, US-A 4,921,843; Kuhl et al., EP-A 0 499 348) vorausgeht. Bei diesen Präparaten wird bei Einnahmebeginn entweder bereits am ersten Zyklustag (Kühl) oder frühestens am zweiten Zyklustag (Pasquale) mit der Einnahme von Dosierungseinheiten begonnen, die nur einen estrogenen Wirkstoff enthalten, und zwar in einer Dosierung, die unter der ovulationshemmenden Dosis der estrogenen Komponente liegt, wodurch es zu Follikelentwicklungen kommen kann. Follikelentwicklungen werden für Durchbruchsovulationen verantwortlich gemacht (Chowdhury et al., "Escape" ovulation in women due to the missing of low dose combination oral contraceptive pills, Contraception, 22: 241-247, 1980; Molloy B.G. et al., "Missed pill" conception: fact or fiction? Brit.Med.J. 290, 1474-1475, 1985). Der kontrazeptive Schutz ist dadurch in Frage gestellt. Das Risiko einer Schwangerschaft ist daher insbesondere bei Einnahmefehlern unter den 20µg-Ethinylestradiol Präparaten hoch.

Die Aufgabe der vorliegenden Erfindung ist es, ein Kombinationspräparat mit möglichst niedrigem Estrogengehalt in jeder einzelnen Dosierungseinheit aber auch mit einem niedrigen Gesamthormongehalt pro Verabreichungszyklus zur Verfügung zu stellen, wobei bei hoher kontrazeptiver Sicherheit eine möglichst geringe Inzidenz an Follikelentwicklung, eine einwandfreie Zykluskontrolle unter zuverlässiger Vermeidung von Zwischenblutungen wie Durchbruchblutungen und "spottings" sowie möglichst wenig Amenorrhoen erreicht und unerwünschte Nebenwirkungen vermieden werden sollen.

Diese Aufgabe wird durch die Bereitstellung des eingangs angegebenen zweiphasischen Kombinationspräparates, in welchem sich zwischen der ersten und der zweiten Hormonkomponente 2 oder 1 wirkstofffreie Tageseinheiten (Placebos) befinden oder 2 oder 1 Blindpillen-Tage angezeigt sind gelöst.

In der ersten Phase wird beginnend mit dem ersten Zyklustag eine Dosierungseinheit enthaltend ein Estrogen in Kombination mit einer gestagenen Komponente täglich über 23 oder 24 Tage verabreicht. Im Anschluß an diese 23 oder 24 täglichen Dosierungseinheiten erfolgt die Verabreichung von 2 oder 1 wirkstofffreien Tageseinheiten oder es werden 2 oder 1 Blindpillen-Tage angezeigt. Daran schliesst sich die zweite Phase an, in der über den verbleibenden Zeitraum zum vorzugsweise 28 Tage umfassenden Zyklus über 4, 3 oder 2 Tage anschließend ein Estrogen verabreicht wird.
Die erste, sowohl Estrogen- als auch Gestagen-haltige Phase kann dabei auch in dem Fachmann geläufiger Weise mehrstufig ausgebildet sein.

Bei Einnahme des erfindungsgemäßen Kombinationspräparates wird bereits im ersten Verabreichungszyklus die Rekrutierung des dominanten Follikels, die im spontanen Zyklus während der ersten 6 Tage des Menstruationszyklus erfolgt, effizient unterdrückt. Somit lassen sich mit dem Kombinationspräparat der vorliegenden Erfindung die Follikelentwicklung bereits im ersten Einnahmezyklus unterdrücken und damit Durchbruchsovulationen vermeiden, wodurch die kontrazeptive Sicherheit erhöht wird.
Dies ist vor allem bei Einnahmefehlern von eminenter Wichtigkeit, und zwar insbesondere bei hormonalen Kontrazeptiva mit niedriger täglicher Ethinylestradiol-Dosismenge. Da bei 25% der Frauen, die die Pille nehmen, Einnahmefehler (Auslassen von Dosierungseinheiten oder Verlängerung des Intervalls auf über 24 Stunden zwischen der täglichen Einnahme zweier Dosierungseinheiten) bekannt sind (Finlay I.G., Scott M.B.G.: Pattems of contraceptive pill-taking in an inner city practice. Br.Med.J. 1986, 293: 601-602), erhöht das erfindungsgemäße Kombinatioinspräparat, wenn es als ovulationshemmendes Mittel verwendet wird, die kontrazeptive Sicherheit. Dies trifft insbesondere bei niedrigst dosierten Präparaten zu.
Die Erhöhung der Anzahl der sowohl Estrogen- als auch Gestagen- haltigen Dosierungseinheiten über die übliche Zahl von 21 Tagen auf 23 oder 24 Tage bewirkt eine effektvolle Verkürzung des pillenfreien Intervalls, in dem bei konventionellen Kombinationspräparaten wie in einem normalen Menstruationszyklus die Selektion von Follikeln erfolgt und somit eine Follikelentwicklung entsteht und vermehrt endogenes Estrogen gebildet wird. Diese Follikel führen, wie schon oben ausgeführt, zu Durchbruchsovulationen. Besonders bei Einnahmefehlern treten diese Durchbruchsovulationen vermehrt auf.
Die anschließende Verabreichung von 2 oder 1 hormonfreien Tageseinheiten beziehungsweise Einfügung von 2 oder 1 Blindpillen-Tagen sowie die nachfolgende Phase, in der über 4, 3 oder 2 Tage Dosierungseinheiten täglich verabreicht werden, die nur eine estrogene Komponente als hormonalen Wirkstoff enthalten, gewährleistet eine Abbruchblutung und bewirkt im folgenden Verabreichungszyklus eine verringerte Zwischenblutungsrate verglichen mit herkömmlichen, niedrig dosierten Präparaten erreicht wird.

Gemäß einer bevorzugten Ausführungsform der Erfindung wird das Estrogen der ersten Hormonkomponente ausgewählt aus der Gruppe der Verbindungen
17β-Estradiol,
Ethinylestradiol und
17β-Estradiolvalerat
und das Gestagen ausgewählt aus der Gruppe der Verbindungen
Gestoden,
Levonorgestrel,
Desogestrel,
3-Ketodesogestrel,
Drospironenon,
Cyproteronacetat,
Norgestimat und
Norethisteron sowie
das Estrogen der zweiten Hormonkomponente ausgewählt aus der Gruppe der Verbindungen
17β-Estradiol,
Ethinylestradiol und
17β-Estradiolvalerat.

Gemäß einer weiteren bevorzugten Variante der vorliegenden Erfindung ist das Estrogen der ersten Hormonkomponente in jeder täglichen Dosierungseinheit in einer Dosis von
1.0 bis 6.0 mg 17β-Estradiol,
0.015 bis 0.025 mg Ethinylestradiol,
1.0 bis 4.0 mg 17β-Estradiolvalerat
und das Gestagen in jeder täglichen Dosierungseinheit in einer Dosis von
0.05 bis 0.075 mg Gestoden,
0.05 bis 0.125 mg Levonorgestrel,
0.06 bis 0.15 mg Desogestrel,
0.06 bis 0.15 mg 3-Ketodesogestrel,
1.0 bis 3.0 mg Drospironenon,
1.0 bis 2.0 mg Cyproteronacetat,
0.2 mg bis 0.3 mg Norgestimat,
0.35 bis 0.75 mg Norethisteron
enthalten.

Die zweite Hormonkomponente enthält das Estrogen in jeder täglichen Dosierungseinheit vorzugsweise in einer Menge von
1.0 bis 6.0 mg 17β-Estradiol,
0.002 bis 0.04 mg Ethinylestradiol,
1.0 bis 4.0 mg 17β-Estradiolvalerat.

Gemäß einer besonders bevorzugten Ausführungsform enthält die zweite Hormonkomponente als Estrogen Ethinylestradiol in jeder täglichen Dosierungseinheit in einer Menge von 0.01 bis 0.015 mg.

Ein Präparat gemäß vorliegender Erfindung enthält insgesamt vorzugsweise 28 Hormontageseinheiten.

Als Estrogen für die erste sowohl als auch die zweite Hormonkomponente kommt in erster Linie Ethinylestradiol in Betracht.

Von den genannten Gestagenen für die zweite Hormonkomponente ist Gestoden hervorzuheben; auch Levonorgestrel ist bevorzugt.

17β-Estradiolvalerat, welches als Estrogen sowohl in der ersten als auch in der zweiten Hormonkomponente enthalten sein kann, ist nur als ein möglicher Vertreter dieser 17β -Estradiolester genannt; auch andere derartige, homologe Ester können als estrogene Komponente im Rahmen der vorliegenden Erfindung verwendet werden.

Das nachfolgenden Beispiele dienen der näheren Erläuterung der vorliegenden Erfindung:

### Beispiele:

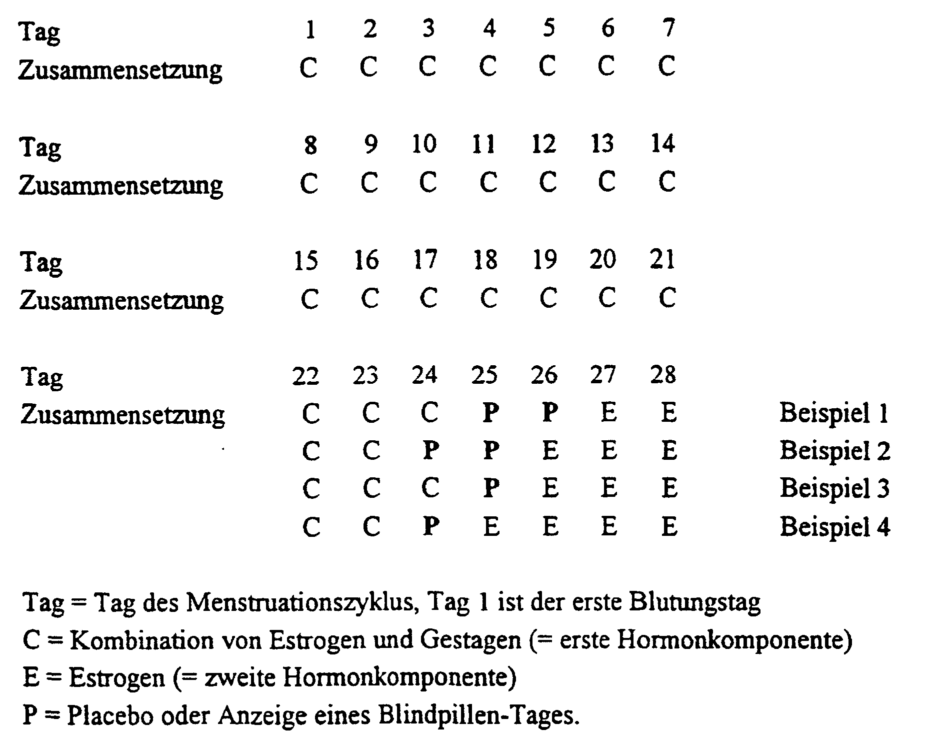

Die Formulierung der Dosierungseinheiten erfolgt konventionell unter Verwendung von fiir die Herstellung Estrogen-/Gestagen- sowie ausschließlich Estrogen-haltiger Tabletten, Pillen, Dragees, etc. bekannter Hilfsstoffe.

Die wirkstofffreien Tageseinheiten werden ausschließlich aus diesen Hilfsstoffen formuliert. Anstelle 2 oder 1 wirkstofffreier Tageseinheiten können in dem erfindungsgemäßen Kombinätionspräparat auch Anzeigen enthalten sein, die (der Anwenderin) signalisieren, daß die Einnahme der ersten Hormonkomponente von einer zwei- oder eintägigen Pause ohne Einnahme jedweder Dosierungseinheit gefolgt sein soll, bevor mit der vier-, drei- oder zeitägigen Einnahme der zweiten Hormonkomponente fortgefahren wird.
Das erfindungsgemäße Kombinationspräparat dient der Empfängnisverhütung für die Frau durch Verabreichung der täglichen Dosierungseinheiten der ersten Hormonkomponente über 23 oder 24 Tage, beginnend am Tag eins des Menstruationszyklus (erster Tag der Menstruationsblutung), sich anschließender zweioder eintägiger Hormonpause, gefolgt von 4, 3 oder 2 täglichen Dosierungseinheiten, die ausschließlich ein Estrogen (E) enthalten, während insgesamt mindestens 28 Tagen im Verabreichungszyklus. Mit diesem Kombinationspräparat kann eine ausgeprägte ovarielle Suppression ohne häufige Follikelanreifung sowie hervorragender Zykluskontrolle bei niedriger täglicher Estrogendosierung, niedriger Gesamtestrogensowie niedriger Gesamthormonmenge pro Verabreichungszyklus erreicht werden.

Die Vorteile dieses über im allgemeinen 28 Tage verabreichten, erfindungsgemäßen Kombinationspräparates (ovulationshemmendes Mittel) gegenüber den bisher beschriebenen Präparaten, insbesondere denen mit einer täglichen Ethinylestradiol-Dosis von weniger als 30µg und solchen mit längeren pillenfreiem Intervall, lassen sich wie folgt charakterisieren:
1. Eine signifikant geringere Häufigkeit von Follikelentwicklungen bei der Anwenderin. Dies bedeutet eine geringere Gefahr von Durchbruchovulationen und damit eine grössere kontrazeptive Zuverlässigkeit insbesondere bei Einnahmefehlern.
2. Die Rekrutierung des dominanten Follikels wird durch die Verlängerung der Einnahme der Kombination auf 23 oder 24 Tage bereits im ersten Zyklus unterdrückt.
3. Die Einnahme von je 4, 3 oder 2 täglichen Estrogen-Dosierungseinheiten im Anschluss an die Verabreichung der 23 oder 24tägigen Kombinationsdosierung und die zwei oder eintägige Pause führt zu einer deutlich verbesserten Zykluskontrolle und zu einer geringeren Inzidenz von Nebenwirkungen wie Kopfschmerzen im Rahmen des prämenstruellen Syndroms.
4. Andere klinische Symptome, die auf stark fluktuierende endogene Estrogenspiegel zurückzuführen sind, wie beispielsweise Brustspannen, sind aufgrund der bedeutend stärkeren ovariellen Supprimierung ebenfalls deutlich verringert.
5. Es kommt zu einer besseren Zykluskontrolle, und zwar vom ersten Einahmezyklus an. Durch die 1 beziehungsweise 2-tägige Einnahmepause im Anschluss an die Verabreichung der 23 oder 24-tägigen Kombinationsdosierung und vor der Einnahme von je 4, 3 oder 2 täglichen Estrogendosierungseinheiten wird eine zuverlässige Abbruchblutung gewährleistet und somit die Amenorrhoerate verringert.
6. Die verbesserte Zykluskontrolle und die sehr geringe Amenorrhoe-Inzidenz führt zu einer höheren Compliance.

Die Formulierung eines Estrogens und Gestagens fiir die Herstellung eines erfindungsgemäßen Kombinationspräparates erfolgt vollkommen analog, wie es bereits fiir herkömmliche orale Kontrazeptiva mit 21tägiger Einnahmedauer der Wirkstoffe, wie beispielsweise Femovan® (Ethinylestradiol/Gestoden) oder Microgynon® (Ethinylestradiol/Levonorgestrel) bekannt ist. Die Formulierung der ausschließlich Estrogen-haltigen Dosierungseinheiten kann ebenso ganz analog wie es fiir schon erhältliche, zur oralen Anwendung bestimmten Estrogen-haltige Mittel, beispielsweise ProgynonC®, bekannt ist, durchgeführt werden.
Eine ein erfindungsgemäßes Kombinationspräparat enthaltende Packung ist ebenfalls analog wie Packungen für bereits bekannte, am Markt befindliche orale Kontrazeptiva aufgebaut, mit der Abweichung, daß anstelle der üblichen 21, die aktiven Bestandteile enthaltenden, Dosierungseinheiten, nunmehr 23 oder 24 derartige Dosierungseinheiten, welche durch 2 oder 1 wirkstofffreie Tageseinheiteiten oder 2 oder 1 Blindpillen-Tagen, die angezeigtwerden, weitere 4, 3 oder 2 lediglich Estrogen-haltige Dosierungseinheiten, vorhanden sind. Als Verpackungsform fiir das erfindungsgemäße Kombinationspräparat dient im allgemeinen eine herkömmliche Blisterpackung, jedoch sind auch andere fiir diesen Zweck bekannte Verpackungsformen denkbar.

Zur Bestimmung wirkequivalenter Mengen von Ethinylestradiol und 17β-Estradiol einerseits und verschiedener Gestagene wie Gestoden, Levonorgestrel, Desogestrel und 3-Ketodesogestrel andererseits wird auf die in der EP-A- 0 253 607 gemachten Angaben verwiesen. Weitere Einzelheiten zur Bestimmung von Dosisequivalenten verschiedener gestagener Wirkstoffe finden sich beispielsweise in "Probleme der Dosisfindung: Sexualhormone"; F. Neumann et al. in "Arzneimittelforschung" (Drug Research) 27, 2a, 296 - 318 (1977) sowie in "Aktuelle Entwicklungen in der homonalen Kontrazeption"; H. Kühl in "Gynäkologe" 25: 231- 240 (1992).

## Patentansprüche

1. Pharmazeutisches Kombinationspräparat mit zwei in einer Verpackungseinheit räumlich getrennt konfektionierten, zur zeitlich sequentiellen oralen Verabreichung bestimmten Hormonkomponenten, die jeweils aus einer Anzahl räumlich getrennt und einzeln entnehmbar in der Verpackungseinheit untergebrachten täglichen Dosierungseinheiten bestehen, wobei eine erste der Hormonkomponenten als hormonellen Wirkstoff in Kombination ein Estrogen- und in mindestens zur Ovulationshemmung ausreichender Dosierung ein Gestagenpräparat in entweder einoder mehrstufiger Ausbildung und die zweite Hormonkomponente als hormonellen Wirkstoff lediglich ein Estrogenpräparat enthält, wobei die erste Hormonkomponente 23 oder 24 und die zweite Hormonkomponente 4, 3 oder 2 Tageseinheiten umfaßt und sich zwischen diesen beiden Hormonkomponenten 2 oder 1 wirkstofffreie Tageseinheiten (Placebos) befinden oder 2 oder 1 Blindpillen-Tage angezeigt sind und die Gesamtzahl der Hormontageseinheiten gleich der Gesamtzahl der Tage des gewünschten, mindestens aber 28 Tage langen, Zyklus ist.

2. Kombinationspräparat nach Anspruch 1, **dadurch gekennzeichnet, daß** das Estrogen der ersten Hormonkomponente aus der Gruppe der Verbindungen
17β-Estradiol,
Ethinylestradiol und
17β-Estradiolvalerat
und das Gestagen aus der Gruppe der Verbindungen
Gestoden,
Levonorgestrel,
Desogestrel,
3-Ketodesogestrel,
Drospironenon,
Cyproteronacetat,
Norgestimat und
Norethisteron sowie
das Estrogen der zweiten Hormonkomponente aus der Gruppe der Verbindungen.
17β-Estradiol,
Ethinylestradiol und
17β-Estradiolvalerat
ausgewählt ist

3. Kombinationspräparat nach Anspruch 2, **dadurch gekennzeichnet, daß** das Estrogen der ersten Hormonkomponente in jeder täglichen Dosierungseinheit in einer Dosis von
1.0 bis 6.0 mg 17β-Estradiol,
0.015 bis 0.025 mg Ethinylestradiol,
1.0 bis 4.0 mg 17β-Estradiolvalerat
und das Gestagen in jeder täglichen Dosierungseinheit in einer Dosis von
0.05 bis 0.075 mg Gestoden
0.05 bis 0.125 mg Levonorgestrel
0.06 bis 0.15 mg Desogestrel
0.06 bis 0.15 mg 3-Ketodesogestrel
1.0 bis 3.0 mg Drospironenon
1.0 bis 2.0 mg Cyproteronacetat
0.2 mg bis 0.3 mg Norgestimat
0.35 bis 0.75 mg Norethisteron
enthalten ist.

4. Kombinationspräparat nach Anspruch 2 oder 3, **dadurch gekennzeichnet, daß** in der zweiten Hormonkomponente in jeder täglichen Dosierungseinheit eine Menge von
1.0 bis 6.0 mg 17β-Estradiol,
0.002 bis 0.04 mg Ethinylestradiol,
1.0 bis 4.0 mg 17β-Estradiolvalerat.
enthalten ist.

5. Kombinationspräparat nach Anspruch 4, **dadurch gekennzeichnet, daß** in der zweiten Hormonkomponente in jeder täglichen Dosierungseinheit Ethinylestradiol in einer Menge von 0.01 bis 0.015 mg enthalten ist.

6. Kombinationspräparat nach einem der vorhergehenden Ansprüche, **dadurch gekennzeichnet, daß** die Gesamtzahl der Hormontageseinheiten sowie der wirkstofffireien Tageseinheiten beziehungsweise der Blindpillen-Tage 28 beträgt.

## Claims

1. Pharmaceutical combination preparation with two hormone components that are packaged spatially separately in a packaging unit and that are intended for time-sequential oral administration, which in each case consist of a number of daily dosage units that are placed spatially separately and individually removably in the packaging unit, where as a hormonal active ingredient a first hormone component contains in combination an estrogen preparation and in at least a dosage that is sufficient to inhibit ovulation a progestogen preparation in either a one-phase or multiple-phase form and as a hormonal active ingredient the second hormone component contains only an estrogen preparation, where the first hormone component comprises 23 or 24 daily units and the second hormone component comprises 4, 3 or 2 daily units, and between these two hormone components there are 2 or 1 active ingredient-free daily units (placebos), or 2 or 1 blank pill days are indicated, and the total number of hormone daily units is equal to the total number of days of the desired cycle, but at least 28 days in length.

2. Combination preparation according to Claim 1, **characterized in that** the estrogen of the first hormone component is selected from the group of compounds
17β-estradiol,
ethinylestradiol and
17β-estradiol valerate
and the progestogen is selected from the group of compounds
gestodene,
levonorgestrel,
desogestrel,
3-ketodesogestrel,
drospironenone,
cyproterone acetate,
norgestimate and
norethisterone and
the estrogen of the second hormone component is selected from the group of compounds
17β-estradiol,
ethinylestradiol and
17β-estradiol valerate.

3. Combination preparation according to Claim 2, **characterized in that** the estrogen of the first hormone component is contained in each daily dosage unit in a dose of
1.0 to 6.0 mg of 17β-estradiol,
0.015 to 0.025 mg of ethinylestradiol, and
1.0 to 4.0 mg of 17β-estradiol valerate
and the progestogen is contained in each daily dosage unit in a dose of
0.05 to 0.075 mg of gestodene,
0.05 to 0.125 mg of levonorgestrel,
0.06 to 0.15 mg of desogestrel,
0.06 to 0.15 mg of 3-ketodesogestrel,
1.0 to 3.0 mg of drospironenone,
1.0 to 2.0 mg of cyproterone acetate,
0.2 mg to 0.3 mg of norgestimate and
0.35 to 0.75 mg of norethisterone.

4. Combination preparation according to Claim 2 or 3, **characterized in that** the second hormone component contains, in each daily dosage unit, an amount of:
1.0 to 6.0 mg of 17β-estradiol,
0.002 to 0.04 mg of ethinylestradiol, and
1.0 to 4.0 mg of 17β-estradiol valerate.

5. Combination preparation according to Claim 4, **characterized in that** the second hormone component in each daily dosage unit contains ethinylestradiol in an amount of 0.01 to 0.015 mg.

6. Combination preparation according to one of the preceding claims, **characterized in that** the total number of hormone daily units and of active ingredient-free daily units or blank pill days is 28.

## Revendications

1. Préparation de combinaison pharmaceutique ayant deux composants hormonaux, destinés à l'administration orale séquentielle dans les temps, conditionnés sous une forme séparée dans l'espace dans une unité d'emballage, qui se composent à chaque fois d'un certain nombre d'unités de dosage journalières logées dans l'unité d'emballage, séparées dans l'espace et pouvant être prélevées individuellement, un premier composant des composants hormonaux contenant, en tant qu'agent actif hormonal, en combinaison, une préparation d'estrogène et, sous une forme à une ou plusieurs étapes, dans un dosage suffisant au moins à l'inhibition de l'ovulation, une préparation de progestatif, et le deuxième composant hormonal contenant, en tant qu'agent actif hormonal, simplement une préparation d'estrogène, le premier composant hormonal comprenant 23 ou 24 et le deuxième composant hormonal comprenant 4, 3 ou 2 unités journalières, et entre ces deux composants hormonaux se trouvant 2 ou 1 unités journalières exemptes d'agents actifs (placebos) ou 2 ou 1 journées à pilules aveugles étant indiquées et le nombre total des unités hormonales étant égal au nombre total de journées du cycle souhaité, d'une longueur, toutefois, d'au moins 28 jours.

2. Préparation de combinaison selon la revendication 1, **caractérisée en ce que** l'estrogène du premier composant hormonal est sélectionné parmi le groupe des composés
17β-estradiol,
éthinylestradiol et
valérianate de 17β-estradiol
et **en ce que** le progestatif l'est parmi le groupe des composés
Gestoden,
Lévonorgestrol,
Desogestrel,
3-cétodesogestrel,
drospironénone,
acétate de cyprotérone,
Norgestimat et
Norethisterone
et **en ce que** l'estrogène du deuxième composant hormonal est sélectionné parmi le groupe des composés
17β-estradiol,
éthinylestradiol et
valérianate de 17β-estradiol.

3. Préparation de combinaison selon la revendication 2, **caractérisée en ce que** l'estrogène du premier composant hormonal est contenu dans chaque unité de dosage journalière dans une dose
de 1,0 à 6,0 mg de 17β-estradiol,
de 0,015 à 0,025 mg d'éthinylestradiol,
de 1,0 à 4,0 mg de valérianate de 17β-estradiol
et **en ce que** le progestatif est contenu dans chaque unité de dosage journalière dans une dose
de 0,05 à 0,075 mg de Gestoden,
de 0,05 à 0,125 mg de Lévonorgestrel,
de 0,06 à 0,15 mg de Desogestrel,
de 0,06 à 0,15 mg de 3-cétodesogestrel,
de 1,0 à 3,0 mg de drospironénone,
de 1,0 à 2,0 mg d'acétate de cyprotérone,
de 0,2 mg à 0,3 mg de Norgestimat,
de 0,35 à 0,75 mg de Norethisterone.

4. Préparation de combinaison selon la revendication 2 ou 3, **caractérisée en ce que**, dans le deuxième composant hormonal dans chaque unité de dosage journalière, est contenue une quantité
de 1,0 à 6,0 mg de 17β-estradiol,
de 0,002 à 0,04 mg d'éthinylestradiol,
de 1,0 à 4,0 mg de valérianate de 17β-estradiol.

5. Préparation de combinaison selon la revendication 4, **caractérisée en ce que**, dans le deuxième composant hormonal dans chaque unité de dosage journalière, de l'éthinylestradiol est contenu dans une quantité de 0,01 à 0,015 mg.

6. Préparation de combinaison selon l'une quelconque des revendications précédentes, **caractérisée en ce que** le nombre total des unités journalières hormonales ainsi que des unités journalières exemptes d'agents actifs, respectivement des journées à pilules aveugles, est de 28.
